# EUROPÄISCHE PATENTSCHRIFT

(11) **EP 0 570 405 B1**
(45) Veröffentlichungstag und Bekanntmachung des Hinweises auf die Patenterteilung: **24.05.1995**
(21) Anmeldenummer: 92903592.1
(22) Anmeldetag: 04.02.1992
(51) Int. Cl.: A61K 7/48

(54) **STABILE KOSMETISCHE MITTEL**
STABLE COSMETIC PREPARATIONS
PRODUITS COSMETIQUES STABLES

(30) Priorität: 06.02.1991 DE 4103557; 23.01.1992 DE 4201694
(43) Veröffentlichungstag der Anmeldung: 24.11.1993
(73) Patentinhaber: Beiersdorf Aktiengesellschaft, 20245 Hamburg (DE)
(72) Erfinder: KUSCHEL, Annegret, D-2070 Ahrensburg (DE); MEIRING, Uta, D-2000 Hamburg 20 (DE); PAPE, Wolfgang, D-2000 Hamburg 20 (DE); SCHNEIDER, Günther, D-2000 Hamburg 20 (DE)
(86) Internationale Anmeldenummer: DE9200066
(87) Internationale Veröffentlichungsnummer: WO9213518

(56) Entgegenhaltungen:
- EP-A- 0 388 275
- DE-A- 3 524 788
- FR-A- 1 343 753
- US-A- 4 201 235
- WORLD PATENTS INDEX LATEST Section Ch, Week 8630, Derwent Publications Ltd., London, GB; Class A, AN 86-195004

## Beschreibung

Die vorliegende Erfindung betrifft stabile kosmetische Mittel, insbesondere Wasser-in-Öl-Emulsionen, welche gegen physikalische Zersetzung geschützt sind.

Unter Hautpflege im Sinne der Erfindung ist in erster Linie zu verstehen, daß die natürliche Funktion der Haut als Barriere gegen Umwelteinflüsse (z.B. Schmutz, Fremdstoffe, Mikroorganismen) und gegen den Verlust von körpereigenen Stoffen (z.B. Wasser, Elektrolyte, natürliche feuchtigkeitsbindende Stoffe) gestärkt, unterstützt und, falls nötig, wiederhergestellt wird.

Wird die natürliche Funktion der Haut gestört, kann es zu verstärkter Resorption toxischer oder allergener Fremdstoffe oder zum Befall mit pathogenen Mikroorganismen und als Folge zu toxischen oder allergischen Hautreaktionen kommen.

Die menschliche Haut verliert durch körpereigene Mechanismen, ebenso wie durch die Transpiration ständig eine gewisse Menge an Feuchtigkeit. Aber auch durch äußere Einflüsse wie die tägliche Körperwäsche, Wind und Wetter verliert die Haut wichtige funktionale Bestandteile.

Obwohl die gesunde Haut durchaus imstande ist, diesen Verlust auszugleichen, ist es Ziel der Hautpflege, die Haut beim Ausgleich dieses Verlustes zu unterstützen. Gerade dann aber, wenn das natürliche Regenerationsvermögen, zum Beispiel infolge starker Belastung oder gar einer Erkrankung, nicht ausreicht, ist die Regulation der Hautfeuchtigkeit bzw. anderer relevanter Inhaltsstoffe unerläßlich.

Folgende Methoden der Hautpflege sind an sich bekannt:
a) Okklusion der Haut
   Wird die Haut mit einem Lipid- oder Lipid/Wasser-Film (herkömmliche Salben oder Crèmes) bedeckt, wird die Barrierefunktion der Haut nicht wiederhergestellt. Der Lipidfilm stellt nur eine äußere physikalische Schutzschicht dar, die zwar verhindert, daß die Haut austrocknet. Mit Reinigungsmitteln kann dieser Film aber leicht abgewaschen werden, so daß wieder der ursprüngliche, beeinträchtigte Zustand erreicht ist.
b) Behandlung der Haut mit essentiellen Fettsäuren Essentielle Fettsäuren werden derzeit gelegentlich in dermatologischen Präparaten zur Behandlung von trockener Haut eingesetzt. Eindeutige Beweise zu deren Wirksamkeit stehen aber noch aus.
c) Behandlung der Haut mit keratolytisch wirksamen Substanzen (z.B. Harnstoff, Salicylsäure, Hydroxycarbonsäuren usw.). Diese Substanzen wirken je nach verwendeter Konzentration keratolytisch, proteolytisch, wasserbindend, penetrationsfördernd, epidermisverdünnend oder juckreizstillend. Ihre Verwendung ist im wesentlichen auf medizinische Indikationen beschränkt.
d) Behandlung mit feuchtigkeitsregulierenden Substanzen (z.B. Glycerin, Sorbit usw.) Die Gruppe dieser Substanzen ist von besonderem Interesse, da sie viele gut wirksame Vertreter enthält. Nachteilig ist indes, daß die meisten dieser Stoffe entweder kostspielig oder aber nur unter aufwendigen Bedingungen in kosmetische Zubereitungen einzuarbeiten sind. Zudem gilt für manche Vertreter dieser Gruppe das unter Punkt c) Gesagte.

Aufgabe der vorliegenden Erfindung war es also, die Nachteile des Standes der Technik zu beseitigen. Insbesondere sollten hautpflegende Zubereitungen zur Verfügung gestellt werden, welche den durch die tägliche Reinigung der Haut und andere äußere Einflüsse verursachten Feuchtigkeitsverlust der Haut ausgleichen, zumal dann, wenn die natürliche Regeneration nicht ausreicht. Weiterhin soll die Haut vor Umwelteinflüssen wie Sonne und Wind geschützt werden.

Ziel der Hautpflege ist die Wiederherstellung und Förderung eines normalen Funktionsbildes der Haut und der Hautanhangsgebilde.

Ganz besonders nachteilig an vielen Wasser-in-Öl-Emulsionen des Standes der Technik ist ferner, daß sie nicht stabil gegen physikalische Zersetzung sind. Üblicherweise bezeichnet man das am häufigsten auftretende unerwünschte Verhalten als "Ausölen". Dies besagt, daß sich Wasserphase und Ölphase allmählich trennen. Dies äußert sich beispielsweise darin, daß aus einer W/O-Crème (= Wasser-in-Öl-Crème) Öltropfen austreten. Beginnt aber eine Emulsion erst einmal, sich zu zersetzen, so ist dieser Vorgang nicht mit einfachen Mitteln wieder rückgängig zu machen. Zumindest stehen dem Verbraucher solcher Mittel nicht zur Verfügung.

Aufgabe der vorliegenden Erfindung war es also auch, die Nachteile des Standes der Technik in dieser Hinsicht zu beseitigen. Insbesondere sollten stabile hautpflegende kosmetische Mittel zur Verfügung gestellt werden.

Es hat sich überraschenderweise gezeigt, und darin liegt die Lösung der Aufgabe, daß W/O-Emulsionen, enthaltend Gemische aus
(a) wasserlöslichen, pharmazeutisch und/oder kosmetisch unbedenklichen Zinksalzen und
(b) Glycin und/oder
(c) einem oder mehreren Zinksalzen oder Komplexen von Glycin
den Nachteilen des Standes der Technik abhelfen und gegen physikalische Zersetzung geschützt sind.

Erfindungsgemäß ist also die Verwendung von Gemischen aus
(a) wasserlöslichen, pharmazeutisch und/oder kosmetisch unbedenklichen Zinksalzen und
(b) Glycin und/oder
(c) einem oder mehreren Zinksalzen oder Komplexen von Glycin
als gegen physikalische Zersetzung schützendes Prinzip in W/O-Emulsionen.

Die DE-A-35 24 788 beschreibt zwar intravenös applizierbare Fettemulsionen mit einem fakultativen Gehalt an Glycin und Zinksalzen, welche jedoch stets O/W-Emulsionen darstellen. Diese Schrift konnte somit keinen Hinweis in die Richtung der vorliegenden Erfindung geben.

Im Derwent-Abstract WPIL AN 86-195004 zu RO-87619 werden ebenfalls Zubereitungen mit einem Gehalt an Zink und Glycin beschrieben, es handelt sich jedoch ebenfalls um O/W-Emulsionen. Auch diese Schrift konnte somit keinen Hinweis in die Richtung der vorliegenden Erfindung geben.

Die Zinksalze werden gewählt aus der Gruppe der wasserlöslichen pharmazeutisch und kosmetisch unbedenklichen Salze. Einbezogen in diese Gruppe ist die Gruppe der Zinkkomplexe. Es ist auch möglich und gegebenenfalls von Vorteil, statt der Kombination von Glycin und Zinksalzen Zinksalze oder Zinkkomplexe von Glycin einzusetzen.

Besonders bevorzugt ist Zinksulfat.

Die erfindungsgemäßen Zusammensetzungen sind nach derzeitiger Kenntnis absolut unbedenklich. Glycin ist ein wertvoller Bestandteil der menschlichen Nahrung. Zink ist ein wichtiges Spurenelement und essentielles Bestandteil der menschlichen Ernährung.

Die erfindungsgemäßen Gemische können allen üblichen pharmazeutischen bzw. kosmetischen W/O-Emulsionen und solchen Emulsionen mit ähnlichen Eigenschaften wie W/O-Emulsionen einverleibt werden, z.B. als multiplen Emulsionen, Milchen, Lotionen, Crèmes usw.

Vorteilhaft enthalten die erfindungsgemäßen Zusammensetzungen
- Zinksalze entsprechend einem Zn²⁺-Gehalt von 0.05 - 10,00 Gewichts-%,
- Glycin in einem Gehalt von 0,05 - 10,00 Gewichts-%,
bezogen auf die Gesamtzusammensetzung.

Besonders vorteilhaft enthalten die erfindungsgemäßen Zusammensetzungen
- Zinksalze entsprechend einem Zn²⁺-Gehalt von 0.10 - 5,00 Gewichts-%,
- Glycin in einem Gehalt von 0,10 - 5,00 Gewichts-%,
bezogen auf die Gesamtzusammensetzung.

Ganz besonders vorteilhaft enthalten die erfindungsgemäßen Zusammensetzungen
- Zinksalze entsprechend einem Zn²⁺-Gehalt von 0.30 - 1,30 Gewichts-%,
- Glycin in einem Gehalt von 0,50 - 2,50 Gewichts-%, bezogen auf die Gesamtzusammensetzung.

Es ist dem Fachmanne natürlich bekannt, daß anspruchsvolle kosmetische Zusammensetzungen zumeist nicht ohne die üblichen Hilfs- und Zusatzstoffe denkbar sind. Darunter zählen beispielsweise Konsistenzgeber, Füllstoffe, Parfum, Farbstoffe, Emulgatoren, zusätzliche Wirkstoffe wie Vitamine oder Proteine, Lichtschutzmittel, Stabilisatoren, Antioxidantien, Insektenrepellentien, Alkohol, Wasser, Salze, proteolytisch oder keratolytisch wirksame Substanzen usw.

Entsprechend können die erfindungsgemäßen Zusammensetzungen, je nach ihrem Aufbau, beispielsweise verwendet werden als Hautschutzcrème, Reinigungsmilch, Sonnenschutzlotion, Nährcrème, Tages- oder Nachtcrème usw. Es ist gegebenenfalls möglich und vorteilhaft, die erfindungsgemäßen Zusammensetzungen als Grundlage für pharmazeutische Formulierungen zu verwenden.

Die folgenden Beispiele sollen die Erfindung näher erläutern, ohne daß aber beabsichtigt ist, die Erfindung auf diese Beispiele zu beschränken. Vielmehr ist der Fachmann aufgrund seines Fachwissens in der Lage, Modifikationen vorzunehmen, die den Bereich der vorliegenden Erfindung nicht verlassen.

Die Mengenangaben in den Beispielen beziehen sich auf Gewichts-%, bezogen auf die Gesamtzusammensetzung.

### Beispiel 1

| W/O-Emulsion I | |
|---|---|
| Polyglyceryl-3-diisostearat | 1,50 |
| PEG-40-Sorbitanperisostearat | 1,50 |
| Wollwachsalkohol | 3,00 |
| Paraffinöl DAB 9 | 14,00 |
| Vaseline | 8,00 |
| Paraffinwachs | 8,00 |
| 2-Octyldodecanol | 8,00 |
| Aluminiumstearat | 0,50 |
| "Blauöl" | 8,00 |
| Butylenglycol-1,3 | 2,50 |
| ZnSO₄ | 0,90 |
| Glycin | 1,00 |
| NaOH (10 %ig) | zum Einstellen auf pH 6,0 |
| Parfum | nach Belieben |
| Wasser VES | auf 100,00 |

### Beispiel 2

| W/O-Emulsion II | |
|---|---|
| Polyglyceryl-3-diisostearat | 2,00 |
| PEG-40-Sorbitanperisostearat | 2,50 |
| Paraffinöl DAB 9 | 7,00 |
| Isopropylpalmitat | 2,00 |
| Ethylhexylisostearat | 10,00 |
| Butylenglycol-1,3 | 2,50 |
| ZnSO₄ | 0,90 |
| Glycin | 1,00 |
| NaOH (10 %ig) | zum Einstellen auf pH 6,0 |
| Parfum | nach Belieben |
| Wasser VES | auf 100,00 |

### Beispiel 3

| W/O-Emulsion III | |
|---|---|
| Polyglyceryl-3-dioleat | 2,80 |
| Paraffinwachs | 3,00 |
| Paraffinöl DAB 9 | 9,50 |
| Cetearyloctanoat | 10,50 |
| Bienenwachs | 2,50 |
| Glycerin | 4,00 |
| ZnSO₄ | 0,90 |
| Glycin | 1,20 |
| NaOH (10 %ig) | zum Einstellen auf pH 6,0 |
| Parfum | nach Belieben |
| Wasser VES | auf 100,00 |

## Patentansprüche

1. W/O-Emulsionen, enthaltend Gemische aus
(a) Zinksalzen und
(b) Glycin und/oder
(c) einem oder mehreren Zinksalzen oder Komplexen von Glycin

2. W/O-Emulsionen nach Anspruch 1, dadurch gekennzeichnet, daß das Zinksalz Zinksulfat ist.

3. W/O-Emulsionen nach Anspruch 1, dadurch gekennzeichnet, daß das Zinksalz und das Glycin als Zinkglycinat vorliegen.

4. W/O-Emulsionen nach Anspruch 1, dadurch gekennzeichnet, daß sie
- Zinksalze entsprechend einem Zn²⁺-Gehalt von 0.05 - 10,00 Gewichts-%,
- Glycin in einem Gehalt von 0,05 - 10,00 Gewichts-%
bezogen auf die Gesamtzusammensetzung, enthalten.

5. W/O-Emulsionen nach Anspruch 1, dadurch gekennzeichnet, daß sie
- Zinksalze entsprechend einem Zn²⁺-Gehalt von 0.10 - 5,00 Gewichts-%,
- Glycin in einem Gehalt von 0,10 - 5,00 Gewichts-%
bezogen auf die Gesamtzusammensetzung, enthalten.

6. W/O-Emulsionen nach Anspruch 1, dadurch gekennzeichnet, daß sie
- Zinksalze entsprechend einem Zn²⁺-Gehalt von 0.30 - 1,30 Gewichts-%,
- Glycin in einem Gehalt von 0,50 - 2,50 Gewichts-%,
bezogen auf die Gesamtzusammensetzung, enthalten.

7. Verwendung von Gemischen aus
(a) Zinksalzen und
(b) Glycin
als gegen physikalische Zersetzung schützendes Prinzip von W/O-Emulsionen.

## Claims

1. W/O emulsions comprising mixtures of
(a) zinc salts and
(b) glycine and/or
(c) one or more zinc salts or complexes of glycine.

2. W/O emulsions according to Claim 1, characterised in that the zinc salt is zinc sulphate.

3. W/O emulsions according to Claim 1, characterised in that the zinc salt and the glycine are present as zinc glycinate.

4. W/O emulsions according to Claim 1, characterised in that they comprise
- zinc salts corresponding to a Zn²⁺ content of 0.05 - 10.00% by weight and
- glycine in a content of 0.05 - 10.00% by weight, based on the total composition.

5. W/O emulsions according to Claim 1, characterised in that they comprise
- zinc salts corresponding to a Zn²⁺ content of 0.10 - 5.00% by weight and
- glycine in a content of 0.10 - 5.00% by weight, based on the total composition.

6. W/O emulsions according to Claim 1, characterised in that they comprise
- zinc salts corresponding to a Zn²⁺ content of 0.30 - 1.30% by weight and
- glycine in a content of 0.50 - 2.50% by weight, based on the total composition.

7. Use of mixtures of
(a) zinc salts and
(b) glycine
as a principle in W/O emulsions which provides protection against physical decomposition.

## Revendications

1. Emulsions eau/huile, contenant des mélanges
(a) de sels de zinc et
(b) de glycine et/ou
(c) d'un ou de plusieurs sels de zinc ou complexes de glycine.

2. Emulsions eau/huile selon la revendication 1, caractérisées en ce que le sel de zinc est le sulfate de zinc.

3. Emulsions eau/huile selon la revendication 1, caractérisées en ce que le sel de zinc et la glycine sont présents en tant que glycinate de zinc.

4. Emulsions eau/huile selon la revendication 1, caractérisées en ce qu'elles contiennent
- des sels de zinc conformément à une teneur en Zn²⁺ de 0,05 - 10,00 % en poids,
- de la glycine dans une teneur de 0,05 - 10,00 % en poids,
par rapport à la composition globale.

5. Emulsions eau/huile selon la revendication 1, caractérisées en ce qu'elles contiennent
- des sels de zinc conformément à une teneur en Zn²⁺ de 0,10 - 5,00 % en poids,
- de la glycine dans une teneur de 0,10 - 5,00 % en poids,
par rapport à la composition globale.

6. Emulsions eau/huile selon la revendication 1, caractérisées en ce qu'elles contiennent
- des sels de zinc conformément à une teneur en Zn²⁺ de 0,30 - 1,30 % en poids,
- de la glycine dans une teneur de 0,50 - 2,50 % en poids,
par rapport à la composition globale.

7. Utilisation de mélanges
(a) de sels de zinc et
(b) de glycine
en tant que principe de protection contre la décomposition physique d'émulsions eau/huile.
